Europäisches Patentamt

European Patent Office

Office européen des brevets

(11) Veröffentlichungsnummer : **0 187 988 B1**

(12) **EUROPÄISCHE PATENTSCHRIFT**

(45) Veröffentlichungstag der Patentschrift :
17.08.88

(51) Int. Cl.⁴ : **G 03 B 42/04**, A 61 B 6/08,
H 05 G 1/26

(21) Anmeldenummer : 85116308.9

(22) Anmeldetag : 20.12.85

(54) **Kassettenaufnahme für Röntgenfilmkassetten.**

(30) Priorität : 11.01.85 DE 8500625 U

(43) Veröffentlichungstag der Anmeldung :
23.07.86 Patentblatt 86/30

(45) Bekanntmachung des Hinweises auf die Patenterteilung : 17.08.88 Patentblatt 88/33

(84) Benannte Vertragsstaaten :
DE FR

(56) Entgegenhaltungen :
DE-A- 1 614 956
FR-A- 2 287 890
US-A- 3 991 316

(73) Patentinhaber : **Siemens Aktiengesellschaft Berlin
und München
Wittelsbacherplatz 2
D-8000 München 2 (DE)**

(72) Erfinder : **Haltrich, Manfred
Burgleite 21
D-8551 Heroldsbach (DE)**

## Beschreibung

Die Erfindung betrifft eine Kassettenaufnahme für Röntgenfilmkassetten mit einem in ihrem Innern liegenden Strahlendetektor.

Eine Kassettenaufnahme dieser Art ist in der DE-OS-24 49 708 beschrieben. Diese Kassettenaufnahme besteht aus einem Gehäuse, in das die Röntgenfilmkassette einschiebbar und an dem der Strahlendetektor mit Hilfe eines verstellbaren Stabes gelagert ist. Die Einstellung des Strahlendetektors erfolgt dabei mit Hilfe von Skalen am Stab und am Kassettengehäuse.

In der Praxis ist nicht immer eine individuelle Einstellung des Strahlendetektors erforderlich. Häufig genügt es, wenn der Strahlendetektor in einer bestimmten Stellung, insbesondere zentrisch hinter der eingelegten Filmkassette, liegt.

Der Erfindung liegt die Aufgabe zugrunde, eine Kassettenaufnahme der eingangs genannten Art so auszubilden, daß bei einfachem Aufbau die Lagerung des Strahlendetektors an einer vorgegebenen Stelle sichergestellt ist.

Diese Aufgabe ist erfindungsgemäß gelöst durch ein einstückiges Kunststoffteil, das mit der Form des Strahlendetektors angepaßten Einformungen versehen ist, die den Strahlendetektor halten. Bei der erfindungsgemäßen Kassettenaufnahme wird der Strahlendetektor in die dafür vorgesehenen Einformungen des ihn aufnehmenden Kassettenteiles eingelegt und ist dann an der gewünschten Stelle hinter der darüber eingelegten und eingerasteten Röntgenfilmkassette fixiert. Die Filmkassette kann in einfacher Weise entfernt werden, wenn in der Kassettenaufnahme Löcher vorgesehen sind, durch die die Filmkassette von Hand wegdrückbar ist.

Der den Strahlendetektor aufnehmende Teil kann dabei ein Tiefziehteil sein.

Die Maße und Gewichte der Kassettenaufnahme mit darin gelagerter Kassette sind dabei nicht wesentlich größer als diejenigen der Kassette selbst. Daher ist eine leichte Handhabung gegeben. Die Herstellungskosten für die Kassettenaufnahme sind gering.

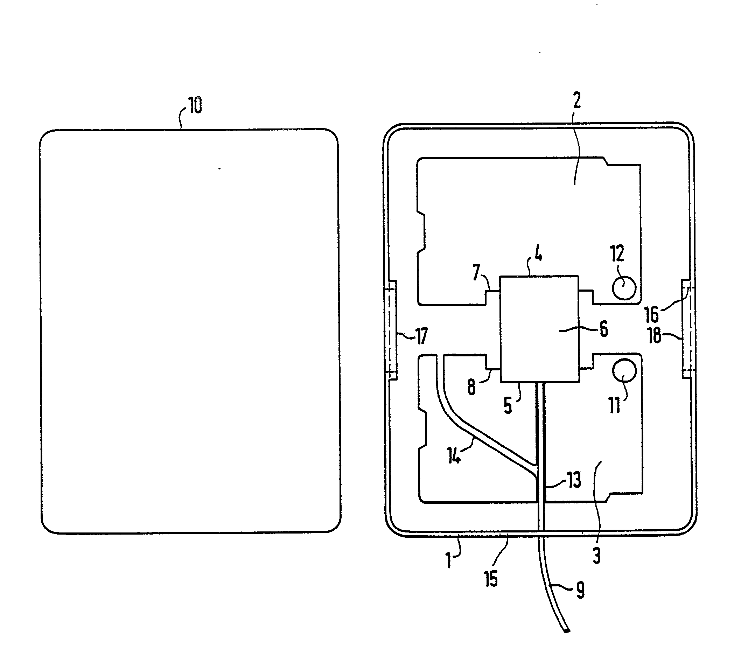

Die Erfindung ist nachfolgend anhand eines in der Zeichnung dargestellten Ausführungsbeispieles näher erläutert.

In der Zeichnung ist eine Kassettenaufnahme 1 dargestellt, die von einem einstückigen Kunststoffziehteil gebildet ist, das Einformungen 2, 3 aufweist, die vom Boden der Kassettenaufnahme 1 aus nach innen vorstehen und die mit Hilfe von Kanten 4, 5 den Platz für einen Strahlendetektor 6 im Hochformat festlegen. Der Strahlendetektor 6 paßt dabei genau zwischen die Kanten 4, 5. Er kann im Querformat eingelegt werden, wenn er an die Kanten 7, 8 angelegt wird. Sein Kabel 9 kann dabei zwischen den Einformungen 2, 3 vorbeigeführt werden, wenn er im Querformat liegt, oder auch in einer dafür vorgesehenen Vertiefung 13 der Einformungen 3 liegen, wie dies in der Zeichnung dargestellt ist (Hochformat). Im Querformat kann das Kabel 9 auch in einer Vertiefung 14 der Einformung 3 liegen, wenn es von der linken Seite des Strahlendetektors 6 ausgeht.

Nach dem Einlegen des Strahlendetektors 6 in der gewünschten Lage wird die Röntgenfilmkassette 10 darübergelegt und durch an der Oberseite der Kassettenaufnahme 1 vorgesehene Ansätze 17, 18 gehalten, die über der Kassette 10 einrasten.

Ein Entfernen der Kassette 10 ist in einfacher Weise möglich, wenn auf diese durch zwei Öffnungen 11, 12 im Boden der Kassettenaufnahme 1 ein leichter Druck ausgeübt wird.

Das Einlegen des Strahlendetektors 6 ist durch zwei passende Schlitze 15, 16 an Längsseiten der Kassettenaufnahme 1 möglich.

## Patentansprüche

1. Kassettenaufnahme für Röntgenfilmkassetten mit einem Strahlendetektor (6), der in ihrem Innern liegt, gekennzeichnet durch ein einstückiges Kunststoffteil (1), das mit der Form des Strahlendetektors (6) angepaßten Einformungen (2, 3) versehen ist, die den Strahlendetektor (6) halten.

2. Kassettenaufnahme nach Anspruch 1, dadurch gekennzeichnet, daß das Kunststoffteil (1) mit Öffnungen (11, 12) versehen ist, durch die die Kassette (10) von Hand abgehoben werden kann.

3. Kassettenaufnahme nach Anspruch 1 oder 2, dadurch gekennzeichnet, daß das den Strahlendetektor (6) aufnehmende Kunststoffteil (1) ein Tiefziehteil ist.

4. Kassettenaufnahme nach einem der Ansprüche 1 bis 3, dadurch gekennzeichnet, daß die Einformungen stufenförmige Kanten (4, 5, 7, 8) begrenzen, die ein Einlegen des rechteckigen Strahlendetektors (6) im Längs- oder Querformat erlauben.

## Claims

1. Cassette receiver, for X-ray film cassettes, having a radiation detector (6) located inside it, characterised by a one-piece synthetic part (1) provided with shaped portions (2, 3) adapted to the shape of the radiation detector (6) and which hold the radiation detector (6).

2. Cassette receiver according to claim 1, characterised in that the synthetic part (1) is provided with openings (11, 12), through which the cassette (10) can be removed by hand.

3. Cassette receiver according to claim 1 or 2, characterised in that the synthetic part (1) which holds the radiation detector (6) is a molded plastic part.

4. Cassette receiver according to one of claims 1 to 3, characterised in that the shaped portions define step-shaped edges (4, 5, 7, 8) which allow the rectangular radiation detector (6) to be inserted lengthwise or crosswise.

## Revendications

1. Logement pour cassette à film radiographique, comportant un détecteur de rayonnement (6) situé à l'intérieur du logement, caractérisé par une pièce en matière plastique d'un seul tenant (1) possédant des parties moulées (2, 3) adaptées à la forme du détecteur de rayonnement (6) et retenant ce dernier.

2. Logement pour cassette suivant la revendication 1, caractérisé par le fait que la pièce en matière plastique (1) comporte des ouvertures (11, 12), grâce auxquelles la cassette (10) peut être retirée à la main.

3. Logement pour cassette suivant la revendication 1 ou 2, caractérisé par le fait que la pièce en matière plastique (1) logeant le détecteur de rayonnement est une pièce emboutie.

4. Logement pour cassette suivant l'une des revendications 1 à 3, caractérisé par le fait que les parties moulées limitent les bords de forme étagée (4, 5, 7, 8), qui permettent l'insertion du détecteur rectangulaire de rayonnement (6) en position longitudinale ou transversale.